# EUROPÄISCHE PATENTANMELDUNG

(11) **EP 2 027 812 A1**
(43) Veröffentlichungstag der Anmeldung: **25.02.2009**
(21) Anmeldenummer: 07114968.6
(22) Anmeldetag: 24.08.2007
(51) Int. Cl.: A61B 5/00

(54) **Verfahren zur Herstellung eines Mikrodialysekatheters und durch dieses hergestellter Mikrodialysekatheter**

(71) Anmelder: F. Hoffmann-La Roche AG, 4070 Basel (CH); Roche Diagnostics GmbH, 68305 Mannheim (DE)
(72) Erfinder: Hochmuth, Gernot, 68165 Mannheim (DE); Mischler, Reinhold, 67063 Ludwigshafen (DE)
(74) Vertreter: Hörschler, Wolfram Johannes

(57) **Zusammenfassung**

Die Erfindung bezieht sich auf einen Mikrodialysekatheter und ein Verfahren zur Herstellung eines Mikrodialysekatheters mit den Schritten
a) Bereitstellen eines Trägers (1), der mindestens einen Leiter (5, 8) zum Zuführen einer Perfusionsflüssigkeit und zum Abführen eines Dialysats enthält und der einen zum Implantieren vorgesehenen Bereich (11) aufweist, in dem mindestens eine fluidisch mit dem Leiter (5, 8) verbundene Öffnung (3) enthalten ist, und
b) Überziehen des Trägers (1) mit einem für einen Analyten per Diffusion durchlässigen, porenfreien Überzug (13), der den zum Implantieren vorgesehenen Bereich (11) von außen zumindest teilweise bedeckt, mit dem Träger (1) stoffschlüssig verbunden ist und die mindestens eine Öffnung (3) überspannt.

## Beschreibung

Die Erfindung bezieht sich auf ein Verfahren zur Herstellung eines Mikrodialysekatheters und auf einen Mikrodialysekatheter. Mikrodialysekatheter dieser Art sind allgemein bekannt und werden dazu eingesetzt, um mit Hilfe der Mikrodialyse bestimmte Parameter im Inneren von biologischem Gewebe (zum Beispiel im Inneren des menschlichen Körpers) zu messen. Ein Beispiel für einen solchen Parameter ist der Gehalt des Blutzuckers im subkutanen Gewebe.

US 6,572,566 B2 bezieht sich auf ein System zur Bestimmung der Konzentration mindestens eines Analyten in einer Körperflüssigkeit. Das System enthält einen Kanal mit einem Austauschbereich, über den Stoffe aus der umgebenden Körperflüssigkeit aufgenommen werden können, und weist weiterhin einen stromabwärts vom Austauschbereich angeordneten Sensor auf, mit dem die Konzentration eines Analyten ermittelt werden kann, wobei das System mindestens ein integriertes Reservoir besitzt, das in Verbindung mit dem Kanal steht. Der Dialysebereich wird von dem nach oben offenen Kanal gebildet, der von einer Membran oder einem perforierten Bereich abgedeckt ist. Wird Perfusionsflüssigkeit durch den Kanal hindurchgeführt, während sich der Austauschbereich in Kontakt mit einer Körperflüssigkeit befindet, so nimmt die Perfusionsflüssigkeit Substanzen aus der Körperflüssigkeit auf. Die Membran oder die perforierte Struktur werden auf den Dialysebereich auf geklebt oder aufgesiegelt. Die Abdeckung einer Kanalstruktur mit bekannten Membranmaterialien bei Erhaltung der porösen Eigenschaften der Membran ist jedoch schwierig.

US 6,632,315 B2 betrifft ein Verfahren zur Herstellung eines Mikrodialysekatheters mit den Schritten
a) Extrudieren eines flexiblen Materials zum Ausbilden eines länglichen Katheterkörpers mit einer im Wesentlichen zylindrischen äußeren Oberfläche und einer Vielzahl von kontinuierlichen inneren Kanälen, die sich in longitudinaler Richtung des Katheterkörpers erstrecken,
b) Versiegeln eines freien Endes des Katheterkörpers,
c) Bereitstellen einer Öffnung in der Oberfläche in einem Abstand von dem freien Ende, die sich in den Katheterkörper erstreckt, und mit einem Teil eines ersten der Kanäle kommuniziert,
d) Bereitstellen einer Kommunikation zwischen dem ersten Kanal und einem zweiten der Kanäle in einem Abstand von dem freien Ende und
e) Abdecken der Öffnung mit einer Mikrodialysemembran.

Die Mikrodialysemembran wird auf den Katheterkörper über die Öffnung geschoben.

US 4,694,832 hat eine Dialysesonde zum Gegenstand, die eine Dialysemembran und Leitungen für den Perfusionsströmungsmittelstrom durch die Membran umfasst. Die Dialysemembran ist von einem Gehäuse umgeben, welches die Membran stützt und teilweise freigibt und darüber hinaus steifer als die Membran selbst ist. Die Leitungen bilden diametral in dem Katheter gegenüberliegende Kanäle, die innerhalb der schlauchförmigen Membran enden. Das verhältnismäßig steife Gehäuse soll verhindern, dass nach Einführen des Katheters in biologisches Gewebe dieses die schlauchförmige Membran zusammendrückt und dadurch deren Funktion und die Strömung innerhalb der schlauchförmigen Membran beeinträchtigt. Dieser bekannte Katheter ist durch die Anbringung des stützenden Gehäuses aufwändig und teuer in der Herstellung und erfordert außerdem einen verhältnismäßig großen Durchmesser, da die Membran in das Gehäuse eingesetzt werden muss.

EP 0 973 573 B1 bezieht sich auf einen Katheter zur Messung chemischer Parameter, insbesondere zum Einführen in biologisches Gewebe, in Flüssigkeiten oder dergleichen. Der Katheter enthält einen Rückführkanal und eine schlauchförmige teildurchlässige Membran. Es sind Distanzmittel in Form von Rippen vorgesehen, die die Innenwandung der schlauchförmgen Membran gegenüber der Außenwandung eines innerhalb der schlauchförmigen Membran angeordneten schlauchförmigen Teils abstützen. Die Distanzmittel unterstützen die Membran jedoch nur in Teilbereichen und sind nicht oder nur an wenigen Stellen mit der Membran verbunden, so dass viele Bereiche der Membran anfällig gegen mechanische Einwirkungen von außen sind.

WO 03/020352 A2 betrifft einen Katheter mit einer im Wesentlichen U-förmig gebogenen Hohlfasermembran, mit zwei Schenkeln und einem gebogenen Bereich, wobei die Hohlfasermembran zumindest über ihren gebogenen Bereich wärmebehandelt und/oder mit mindestens einen Lösemittel behandelt ist, um einen möglichst kleinen Gesamtdurchmesser des Katheters bei durchgängigem Lumen der Hohlfasermembran zu erreichen. Die Hohlfasermembran weist eine Verstärkung auf, wobei als Verstärkung ein Draht, ein Faden und/oder eine Litze vorgesehen und mit den beiden Schenkeln verbunden beziehungsweise verklebt oder verschweißt ist. Auch bei dieser Lösung sind die relativ großen, nicht direkt mit der verstärkenden Struktur verbundenen Bereiche der Hohlfasermembran anfällig gegen mechanische Einwirkungen von außen.

Der Erfindung liegt die Aufgabe zugrunde, ein Verfahren zur Herstellung eines Mikrodialysekatheters und einen Mikrodialysekatheter bereitzustellen, die die Nachteile des Standes der Technik vermeiden. Insbesondere ist es Aufgabe der vorliegenden Erfindung, einen in der Herstellung und der Handhabung unkomplizierten und günstigen Mikrodialysekatheter bereitzustellen, der hinreichend miniaturisierbar ist, um beim Implantieren eine möglichst geringe Wunde zu erzeugen. Der Katheter soll weiterhin mechanisch ausreichend stabil sein, so dass das Risiko, dass beim Explantieren Teile des Katheters im Gewebe verbleiben, minimiert wird.

Die Aufgabe wird erfindungsgemäß gelöst durch ein Verfahren zur Herstellung eines Mikrodialysekatheters, umfassend die Schritte
a) Bereitstellen eines Trägers, der mindestens einen Leiter zum Zuführen einer Perfusionsflüssigkeit und zum Abführen eines Dialysats enthält und der einen zum Implantieren vorgesehenen Bereich aufweist, in dem mindestens eine fluidisch mit dem Leiter verbundene Öffnung enthalten ist, und
b) Überziehen des Trägers mit einem für einen Analyten per Diffusion durchlässigen, porenfreien Überzug, der den zum Implantieren vorgesehenen Bereich von außen zumindest teilweise bedeckt, mit dem Träger stoffschlüssig verbunden ist und die mindestens eine Öffnung überspannt.

Weiterhin wird die Aufgabe erfindungsgemäß gelöst durch einen Mikrodialysekatheter, der vorzugsweise durch ein erfindungsgemäßes Verfahren hergestellt ist, und der einen Träger umfasst, der mindestens einen Leiter zum Zuführen einer Perfusionsflüssigkeit und zum Abführen eines Dialysats enthält, und der einen zum Implantieren vorgesehenen Bereich aufweist, in dem mindestens eine fluidisch mit dem Leiter verbundene Öffnung enthalten ist, wobei der Träger einen für einen Analyten per Diffusion durchlässigen, porenfreien Überzug aufweist, der den zum Implantieren vorgesehenen Bereich zumindest teilweise von außen bedeckt, mit dem Träger stoffschlüssig verbunden ist und die mindestens eine Öffnung überspannt.

Zur Herstellung des erfindungsgemäßen Mikrodialysekatheters wird ein Träger verwendet, der dem Katheter mechanische Stabilität verleiht. Der Träger kann beispielsweise in Form einer Röhre oder eines flachen Trägersubstrats mit einer Abdeckung vorliegen. Vorzugsweise handelt es sich um einen Träger aus Polyurethan (PUR), Polyamid (PA), Polyethylenterephthalat (PET) oder Edelstahl. Diese Materialien sind vorteilhaft in Bezug auf ihre Biokompatibilität, Verarbeitbarkeit und/oder Verfügbarkeit. Falls Röhren oder Schläuche als Träger verwendet werden, so weisen diese vorzugsweise einen Außendurchmesser von 0,5 bis 0,75 mm auf. Bei Verwendung eines flachen Trägersubstrates wird eine Dicke des flachen Trägersubstrats von 0,25 bis 0,5 mm (zuzüglich Dicke der Abdeckung der vorhandenen Überzüge) und eine Breite von 0,5 bis 0,75 mm bevorzugt.

Im Inneren des Trägers ist mindestens ein Leiter zum Zuführen einer Perfusionsflüssigkeit und zum Abführen eines Dialysats angeordnet. Als Leiter können beispielsweise voneinander weitgehend getrennte Innenlumen eines röhrenförmigen Trägers, im Innenraum des Trägers verlaufende Innenröhren oder eine sonstige Kanalstruktur vorgesehen sein. Gemäß einer Ausführungsvariante ist ein U-förmig gebogener röhrenförmiger Leiter in dem Träger vorgesehen. Gemäß einer anderen Ausführungsvariante sind zwei parallel zueinander verlaufende röhrenförmige Leiter vorgesehen, die über Öffnungen im Bereich ihrer distalen Enden fluidisch miteinander verbunden sind. Die distalen Enden sind dabei diejenigen Enden, die in oder nahe einem zum Implantieren vorgesehenen Bereich des Mikrodialysekatheters angeordnet sind. Der zum Implantieren vorgesehene Bereich ist ein Abschnitt des Trägers, der sich im implantierten Zustand des Katheters innerhalb des biologischen Gewebes befindet. Die in dem Träger vorgesehenen röhrenförmigen Leiter können zum Beispiel aus Kunststoff oder Metall bestehen.

In dem zum Implantieren vorgesehenen Bereich des Trägers ist eine fluidisch mit dem mindestens einen Leiter verbundene Öffnung zur Umgebung des Katheters vorgesehen. Durch die Öffnung kann zum Beispiel ein Analyt aus einer Körperflüssigkeit in der Umgebung des implantierten Bereichs in das Innere des Trägers und in eine in dem mindestens einen Leiter enthaltene Flüssigkeit gelangen. Das so erhaltene Dialysat wird über einen Leiter abgeführt und kann dann zum Beispiel mittels eines Sensors in Bezug auf den Analyten untersucht werden.

Der Durchmesser der Öffnung in dem Träger beträgt vorzugsweise zwischen 1 und 10 µm. Die Fläche der Öffnung beträgt vorzugsweise zwischen 1 und 100 µm².

Die mindestens eine Öffnung in dem Träger ist erfindungsgemäß durch einen für einen Analyten per Diffusion durchlässigen, porenfreien Überzug verschlossen, der den zum Implantieren vorgesehenen Bereich von außen zumindest teilweise bedeckt, mit dem Träger stoffschlüssig verbunden ist und die mindestens eine Öffnung überspannt. Der Überzug ist vorzugsweise eine Membran mit einer Dicke von 1 bis 100 µm. Der Überzug ist von dem Analyten (zum Beispiel von Glukose) durch Diffusion durchdringbar und ist nicht porös ausgebildet. Porenfrei bedeutet in diesem Zusammenhang, dass keine mikroskopisch erkennbaren Poren in dem Überzug enthalten sind. Zudem besteht ein Unterschied zu porösen Membranen in der Regel darin, dass der porenfreie Überzug ein dreidimensionales Polymernetzwerk aufweisen kann, welches in einer wässrigen Lösung erheblich quillt, so dass sich das Volumen, in welchem der Analyt sich durch Diffusion bewegen kann, erheblich vergrößern kann. Die Poren einer porösen Membran sind hingegen bereits im trockenen Zustand in der Regel in ihrer Größe uns Form voll ausgebildet. Das Polymernetzwerk der porösen Membran quillt dementsprechend in wässriger Lösung lediglich geringfügig, so dass das im trockenen Zustand vorhandene Porenvolumen annähernd gleich dem durch die wässrige Lösung benetzten Porenvolumen ist. Für weitere Unterschiede zwischen porenfreien, d.h. dichten Membranen und porösen, insbesondere mikroporösen Membranen und Stofftransportmechanismen in beiden Materialarten kann beispielsweise auf US 5,428,123 verwiesen werden.

Im Stand der Technik verwendete Mikrodialysemembran-Materialien weisen im Unterschied zu dem erfindungsgemäß eingesetzten Überzug eine quantifizierbare Porosität im mikroskopischen Maßstab auf. Der Transport des Analyten durch solche mikroporösen Membranen aus dem den Katheter umgebenden Gewebe in das innere Lumen des Katheters erfolgt dabei durch Diffusion durch die in der Membran enthaltenen, mit wässriger Lösung benetzten Poren. Der bei der vorliegenden Erfindung verwendete Überzug ist hingegen ein für einen Analyten permeabler Film, dessen Permeabilität nicht durch mikroskopische Porosität definiert ist.

Besonders bevorzugt dient als Überzug ein Polymer, welches in der Lage ist, gemeinsam mit Wasser kleine Moleküle (wie zum Beispiel Glukose) aufzunehmen und passieren zu lassen und größere Moleküle aufgrund der verschlungenen Polymerstruktur zu sperren. Vorzugsweise weist der Überzug ein hydrophiles Polymer auf oder besteht aus einem hydrophilen Polymer. Beispielsweise kann ein Überzug aus hydrophilem Polyurethan auf dem Träger in dem zum Implantieren vorgesehenen Bereich hergestellt werden. Der Transport eines Analyten durch den Überzug erfolgt durch Diffusion durch die im dreidimensionalen Polymernetzwerk der Membran aus der Umgebung des Trägers aufgenommene wässrige Lösung. Das dreidimensionale Netzwerk der Polymerketten wird durch die wässrige Lösung gequollen, jedoch nicht gelöst.

Der als Membran dienende Überzug kann den Träger in einem Teil des zum Implantieren vorgesehenen Bereichs, in dem gesamten zum Implantieren vorgesehenen Bereich, über den zum Implantieren vorgesehenen Bereich hinausgehend oder den Träger insgesamt bedecken und ist mit diesem stoffschlüssig verbunden. Der Überzug wird erfindungsgemäß zum Überziehen des Trägers direkt auf den Träger abgeschieden. Daher wird der permeable Film auf dem Träger erzeugt und ist mit diesem auf der gesamten von dem Film überspannten Oberfläche des Trägers innig (stoffschlüssig) verbunden.

Der erfindungsgemäße Mikrodialysekatheter hat den Vorteil, dass er den relativ stabilen Träger umfasst, der nur an definierten Stellen mit Öffnungen versehen ist, so dass die mechanische Belastbarkeit des Katheters groß ist. Der als Membran dienende Überzug ist (außer im Bereich der Öffnungen) über seine gesamte Fläche mit dem Substrat verbunden, so dass eine sichere Verbindung vorhanden ist. Der Überzug überspannt die definierten Öffnungen im Träger freitragend. Die nicht unterstützte Fläche des Überzugs ist daher auf die Bereiche der in der Regel sehr kleinen Öffnungen reduziert, so dass die Gefahr einer Beschädigung der Membran minimiert wird.

Zur Herstellung des erfindungsgemäßen Mikrodialysekatheters sind keine komplizierten Montageschritte notwendig, so dass der Katheter gut miniaturisiert hergestellt werden kann. Die Herstellung der Leiter in dem Träger sowie der notwendigen Verbindungen zwischen den Leitern und dem Außenraum kann mit im Stand der Technik gängigen Verfahren erfolgen. Die Erzeugung des Überzugs (Prozess der Membranerzeugung) beruht vorzugsweise auf kontrollierbaren selbstorganisierenden Mechanismen. Beispielsweise kann der Träger in eine Polymerlösung getaucht werden und das Polymer anschließend ausgehärtet werden. Die für diese Verfahren relevanten Parameter sind die Oberflächenspannung und die Viskosität der Polymerlösung.

Gemäß einer bevorzugten Ausführungsform der vorliegenden Erfindung wird der Träger daher in dem zum Implantieren vorgesehenen Bereich mit einer Polymerlösung überzogen, um den Überzug herzustellen. Als Polymerlösung wird eine Lösung bezeichnet, in welcher ein Polymer in einem Lösemittel gelöst vorliegt. Gemäß einer bevorzugten Variante wird der Träger mit einer Lösung von hydrophilem Polyurethan HPU in Ethanol überzogen, um den Überzug herzustellen. Der Überzug bedeckt und überspannt aufgrund der selbstorganisierenden Prozesse bei der Schichterzeugung (aufgrund der Viskosität und/oder der Oberflächenspannung) den Träger beziehungsweise die im Träger enthaltenen Öffnungen.

Vorzugsweise wird der Träger zum Herstellen des Überzugs in eine Polymerlösung getaucht oder mit einer Polymerlösung besprüht, oder der Überzug wird durch Rakeln oder Rotationsbeschichtung mit einer Polymerlösung erzeugt. Das Tauchen des Trägers oder das Aufsprühen der Polymerlösung bietet sich bei beliebig geformten Trägern an. Durch Rakeln oder Rotationsbeschichtung (Spin Coating) können insbesondere Überzüge auf planaren Trägern erzeugt werden.

Vorzugsweise wird der Träger mit einer Polymerlösung überzogen, die anschließend aushärtbar ist, insbesondere die durch Erwärmen, durch Bestrahlen mit elektromagnetischer Strahlung oder durch Kontaktieren mit Wasser ausgehärtet wird. Die polymere Membranschicht wird besonders bevorzugt durch lokales Abdampfen des Lösemittels, in dem das Polymer in gelöster Form vorliegt, erzeugt. Weiterhin sind zum Beispiel eine durch Bestrahlung mit Licht einer geeigneten Wellenlänge, eine durch thermische Einwirkung oder eine durch Kontakt mit Luftfeuchtigkeit initiierte Aushärtereaktion des Polymers möglich.

Gemäß einer bevorzugten Ausführungsform der vorliegenden Erfindung wird der Überzug mit einer Schicht (Deckschicht) aus einem biokompatiblen (porenfreien oder porösen) Material bedeckt. Der Überzug wird daher mit einer weiteren, die Biokompatibilität verbessernden Schicht beschichtet, die von dem Analyten durchströmt werden kann.

Bei einer porenfreien Schicht des biokompatiblen Materials erfolgt der Stofftransport (wie bei dem darunter liegenden Überzug) durch Diffusion. Wesentlich ist bei einer porenfreien biokompatiblen Schicht eine kurze Diffusionszeit des Analyten durch die Schicht. Die Diffusionzeit wird wesentlich durch die Schichtdicke beeinflusst. Die biokompatible Schicht sollte daher möglichst dünn aufgetragen werden. Vorzugsweise beträgt die Dicke der biokompatiblen Schicht zwischen 1 µm und 10 µm. Zur Erzeugung der die Biokompatibilität verbessernden Deckschicht kann zum Beispiel 2-Methacryloyloxyethylphosphorylcholin-Polymer (MPC) verwendet werden, das keine mikroskopische Porosität aufweist.

Der Stofftransport durch eine poröse Schicht des biokompatiblen Materials beruht in der Regel auf einer Diffusion des Analyten innerhalb einer die Poren benetzenden und in der Regel diese auch ausfüllenden wässrigen Lösung. Folglich wird die Durchtrittsgeschwindigkeit des Analyten durch die biokompatible Schicht durch eine Diffusionskonstante definiert. In der Regel ist für die Durchtrittsdauer daher eine geringe Schichtdicke der biokompatiblen Schicht entscheidend, welche vorzugsweise zwischen 1 µm und 10 µm beträgt. Die poröse Schicht kann somit relativ schnell von der Flüssigkeit durchströmt werden und zu dem für den Analyten per Diffusion durchlässigen Überzug gelangen.

Gemäß einer bevorzugten Ausführungsform der vorliegenden Erfindung ist der Träger des Mikrodialysekatheters eine Röhre, deren distales Ende verschlossen ist und die mindestens eine seitliche Öffnung aufweist, die durch einen für einen Analyten per Diffusion durchlässigen, porenfreien Überzug überspannt ist, wobei die Röhre einen longitudinal angeordneten Innenraum enthält, in dem mindestens ein fluidischer Leiter zum Zuführen einer Perfusionsflüssigkeit und zum Abführen eines Dialysats vorgesehen ist, wobei der fluidische Leiter mit der seitlichen Öffnung fluidisch verbunden ist. Der zum Implantieren vorgesehene Bereich umfasst das distale Ende der Röhre. Die mindestens eine seitliche Öffnung ermöglicht das Eintreten einer den Analyten enthaltenden Flüssigkeit aus der Umgebung des Katheters durch den Überzug in den Innenraum der Röhre und dort in den fluidischen Leiter zum Abführen des Dialysats, der mit der seitlichen Öffnung fluidisch verbunden ist. Als fluidische Leiter sind vorzugsweise im Innenraum der Röhre zwei durch eine Trennwand voneinander getrennte Innenlumen oder zwei innere Röhren vorgesehen, die über mindestens eine Öffnung fluidisch miteinander verbunden sind.

Eine mögliche Variante eines röhrenförmigen Trägers mit fluidischen Leitern ist eine Mehrlumenröhre. Die Außenwand einer Mehrlumenröhre, das heißt einer Röhre, in der mehrere (bevorzugt zwei) voneinander durch Trennwände fluidisch getrennte Innenlumen erzeugt wurden, wird in dem Bereich, welcher sich bei der Anwendung des Mikrodialysekatheters innerhalb des Körpers befindet, an der Seite mit Öffnungen versehen, welche eine fluidische Verbindung vom umgebenden Außenraum zu dem Innenraum des Mikrodialysekatheters ermöglichen. Die Trennwand zwischen zwei Innenlumen der Mehrlumenröhre wird kurz vor dem distalen Ende des Mikrodialysekatheters derart strukturiert, dass die beiden Innenlumen fluidisch miteinander verbunden werden. Die Trennwand kann zum Beispiel von der Stirnfläche der Mehrlumenröhre ausgehend mit einem geeigneten Werkzeug (zum Beispiel einem Laser) eingekerbt oder abgetragen werden. Mindestens eines der fluidisch verbundenen Innenlumen wird zur Zuführung einer Perfusionsflüssigkeit genutzt. Mindestens ein weiteres der fluidisch verbundenen Innenlumen wird zur Ableitung des Dialysats genutzt. Die derart strukturierte Mehrlumenröhre wird am distalen Ende gegen den umgebenden Außenraum fluidisch abgedichtet, indem zum Beispiel die Öffnungen an den Enden der Innenlumen mit einer Dichtmasse abgedichtet werden. Die mit den seitlichen Öffnungen sowie mit der fluidischen Verbindung zwischen den Innenlumen und mit der Abdichtung des distalen Endes versehene Röhre wird außen mit einem für den Analyten per Diffusion durchlässigen, porenfreien Überzug versehen. Der Überzug bedeckt die Außenwand der Röhre vorzugsweise vollständig in einem definierten Bereich, welcher sich im implantierten Zustand des Katheters im Gewebe befindet. Der Überzug ist mit der Außenwand verbunden. Die eingebrachten seitlichen Öffnungen werden von dem Überzug überspannt, so dass die Innenlumen durch den Analyten erreicht werden können. Die mit dem genannten Überzug versehene Mehrlumenröhre wird gegebenenfalls mit einem weiteren biokompatiblen Überzug versehen, welcher von dem Analyten durchströmt werden kann.

Gemäß einer weiteren Ausfiihrungsform der vorliegenden Erfindung ist der Träger des Mikrodialysekatheters ein ebenes Substrat mit einer Abdeckung, wobei das Substrat als Leiter eine Kanalstruktur zum Zuführen einer Perfusionsflüssigkeit und zum Abführen eines Dialysats enthält, wobei die Kanalstruktur durch die Abdeckung des ebenen Substrats geschlossen ist, und wobei das Substrat oder die Abdeckung mindestens eine Öffnung aufweist, die durch einen für einen Analyten per Diffusion durchlässigen, porenfreien Überzug überspannt ist und die mit der Kanalstruktur fluidisch verbunden ist. Zur Herstellung eines solchen Mikrodialysekatheters wird in ein ebenes Substrat eine Kanalstruktur eingebracht, welche die Zuleitung einer Perfusionsflüssigkeit und die Ableitung des Dialysats ermöglicht. Die Kanalstruktur wird allseitig abgeschlossen, zum Beispiel durch Abdeckung einer an der Oberfläche des Substrats liegenden Rinne mit einer Abdeckplatte oder Abdeckfolie. In das Substrat und/oder in die abdeckende Platte/Folie werden von außen Öffnungen eingebracht, welche eine fluidische Verbindung zwischen dem den Mikrodialysekatheter umgebenden Außenraum und der Kanalstruktur herstellen. Das mit den Öffnungen versehene Substrat und/oder die mit den Öffnungen versehene Abdeckung werden außen mit einem für den Analyten per Diffusion durchlässigen, porenfreien Überzug versehen. Der Überzug bedeckt die Außenwand des Substrates und der Abdeckung. Die eingebrachten Öffnungen werden von dem Überzug überspannt. Das mit dem genannten Überzug versehene Substrat wird gegebenenfalls mit einem weiteren biokompatiblen Überzug versehen, welcher von dem Analyten durchströmt werden kann.

Anhand der Zeichnung wird die Erfindung nachstehend näher erläutert.

Es zeigen:
- Figur 1: schematisch eine erste Ausführungsform eines erfindungsgemäßen Mikrodialy- sekatheters mit einem röhrenförmigen Träger und zwei inneren Röhren als flui- dische Leiter,
- Figur 2: schematisch eine zweite Ausführungsform eines erfindungsgemäßen Mikrodia- lysekatheters mit einem röhrenförmigen Träger und zwei inneren Röhren als flu- idische Leiter,
- Figuren 3.1 bis 3.3: schematisch eine dritte Ausführungsform eines erfindungsgemäßen Mikrodialy- sekatheters mit einem Träger in Form einer Mehrlumenröhre und
- Figuren 4.1 bis 4.3: schematisch eine vierte Ausführungsform eines erfindungsgemäßen Mikrodialy- sekatheters mit einem Träger in Form eines ebenen Substrats.

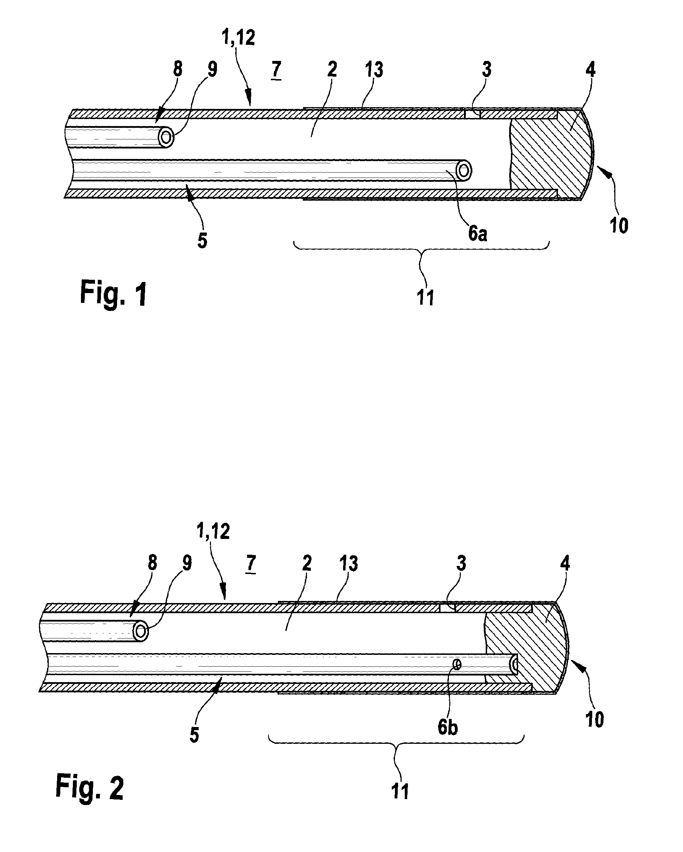

Figur 1 zeigt schematisch einen Ausschnitt aus einem erfindungsgemäßen Mikrodialysekatheter, der nach dem erfindungsgemäßen Verfahren hergestellt wurde.

Der Mikrodialysekatheter umfasst einen Träger 1, der als Röhre 12 ausgebildet ist. In dem longitudinal angeordneten Innenraum 2 der Röhre 12 sind zwei innere Röhren 5, 8 als fluidische Leiter angeordnet. Die erste innere Röhre 5 ist zum Zuführen einer Perfusionsflüssigkeit und die zweite innere Röhre 8 zum Abführen eines Dialysats vorgesehen. Die inneren Röhren 5, 8 können zum Beispiel aus einem Polymer oder einem Metall gefertigt sein. Das distale Ende 10 der Röhre 12 ist durch eine Abdichtung 4 verschlossen und daher fluidisch gegen den umgebenden Außenraum 7 abgedichtet (zum Beispiel mit einer Dichtmasse oder einer Abdeckung).

Der Träger 1 weist einen zum Implantieren vorgesehenen Bereich 11 auf. In diesem Bereich 11 weist die Röhre 12 mindestens eine seitliche Öffnung 3 auf, die über den Innenraum 2 mit den zwei inneren Röhren 5, 8 über deren offene Enden 6a, 9 in fluidischer Verbindung steht. Die erste innere Röhre 5 ragt weiter in die Röhre 12 hinein als die zweite innere Röhre 8, und ihr offenes Ende 6a ist daher näher an der Abdichtung 4 positioniert als das offene Ende 9 der zweiten inneren Röhre 8. Die Flussrichtung eines am (nicht dargestellten) proximalen Ende des ersten fluidischen Leiters (erste innere Röhre 5) eingeleiteten Perfusats erfährt daher, nachdem es aus dem offenen Ende 6a in den Innenraum 2 ausgetreten ist, vor der am distalen Ende 10 des Mikrodialysekatheters befindlichen Abdichtung 4 eine Richtungsumkehr, strömt an der Öffnung 3 vorüber und tritt durch das offene Ende 9 des zweiten fluidischen Leiters 8 in diesen ein.

Der Träger 1 weist einen für einen Analyten per Diffusion durchlässigen, porenfreien Überzug 13 auf, der den zum Implantieren vorgesehenen Bereich 11 von außen bedeckt und mit dem Träger 1 stoffschlüssig verbunden ist. Der Überzug 13 überspannt die mindestens eine Öffnung 3.

Figur 2 zeigt schematisch einen Ausschnitt aus einem weiteren erfindungsgemäßen Mikrodialysekatheter, der nach dem erfindungsgemäßen Verfahren hergestellt wurde.

Der Katheter gemäß Figur 2 ist im Wesentlichen wie der Katheter gemäß Figur 1 aufgebaut, wobei gleiche Bezugszeichen gleiche Bestandteile kennzeichnen. In dieser Ausführungsform ist die erste innere Röhre 5 so ausgeführt, dass sie sich bis in die Dichtmasse der Abdichtung 4 hinein erstreckt und ihr Ende dadurch verschlossen ist. Die erste innere Röhre 5 hat zusätzlich eine seitliche Öffnung 6b nahe dem distalen Ende 10, durch die die Perfusionsflüssigkeit in den Innenraum der Röhre 12 gelangt.

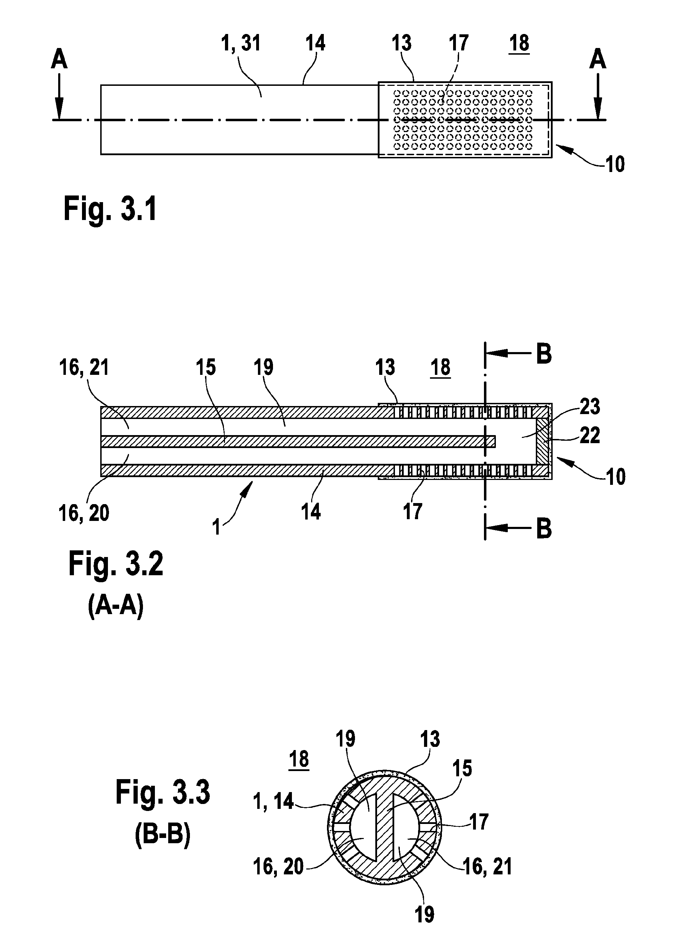

Figuren 3.1 bis 3.3 zeigen schematisch eine dritte Ausführungsform eines erfindungsgemäßen Mikrodialysekatheters mit einem Träger in Form einer Mehrlumenröhre.

Eine mögliche Variante eines röhrenförmigen Trägers 1 mit fluidischen Leitern ist eine Mehrlumenröhre 31. Figur 3.1 zeigt eine Draufsicht, Figur 3.2 einen Längsschnitt (entlang A - A in Figur 3.1) und Figur 3.3 einen Querschnitt (entlang B - B in Figur 3.2) in vergrößerter Darstellung eines solchen Mikrodialysekatheters auf der Basis einer Mehrlumenröhre.

Die röhrenförmige Außenwand 14 der Mehrlumenröhre, das heißt einer Röhre, in der zwei voneinander durch eine Trennwand 15 fluidisch getrennte Innenlumen 16 erzeugt werden, wird in dem Bereich, welcher sich bei der Anwendung des Mikrodialysekatheters innerhalb des Körpers befindet, an der Seite mit Öffnungen 17 versehen, welche eine fluidische Verbindung vom umgebenden Außenraum 18 zu dem Innenraum 19 des Mikrodialysekatheters ermöglichen.

Die Trennwand 15 zwischen den zwei Innenlumen 16 der Mehrlumenröhre wird kurz vor dem distalen Ende 10 des Mikrodialysekatheters derart strukturiert, dass die beiden Innenlumen 16 fluidisch miteinander verbunden werden. Die Trennwand 15 kann zum Beispiel von der Stirnfläche der Mehrlumenröhre ausgehend mit einem geeigneten Werkzeug (zum Beispiel einem Laser) eingekerbt oder abgetragen worden sein. Eines der fluidisch verbundenen Innenlumen 20 wird zur Zuführung einer Perfusionsflüssigkeit genutzt. Das andere der fluidisch verbundenen Innenlumen 21 wird zur Ableitung des Dialysats genutzt.

Die derart strukturierte Mehrlumenröhre ist am distalen Ende 10 gegen den umgebenden Außenraum 18 fluidisch abgedichtet, indem die Öffnungen an den Enden der Innenlumen mit einer Dichtung 22 abgedichtet werden.

Die mit den seitlichen Öffnungen 17 sowie mit der fluidischen Verbindung 23 zwischen den Innenlumen 16 und mit der Abdichtung 22 des distalen Endes 10 versehene Röhre wird außen mit einem für den Analyten per Diffusion durchlässigen, porenfreien Überzug 13 versehen. Der Überzug 13 bedeckt die Außenwand 14 der Röhre in einem definierten Bereich am distalen Ende 10 der Mehrlumenröhre, welcher sich im implantierten Zustand des Katheters im Gewebe befindet. Der Überzug 13 ist mit der Außenwand 14 verbunden. Die eingebrachten seitlichen Öffnungen 17 werden von dem Überzug 13 überspannt, so dass die Innenlumen 16 durch den Analyten erreicht werden können.

Die mit dem genannten Überzug 13 versehene Mehrlumenröhre wird gegebenenfalls mit einer weiteren (nicht dargestellten) biokompatiblen Schicht versehen, welche von dem Analyten durchströmt werden kann.

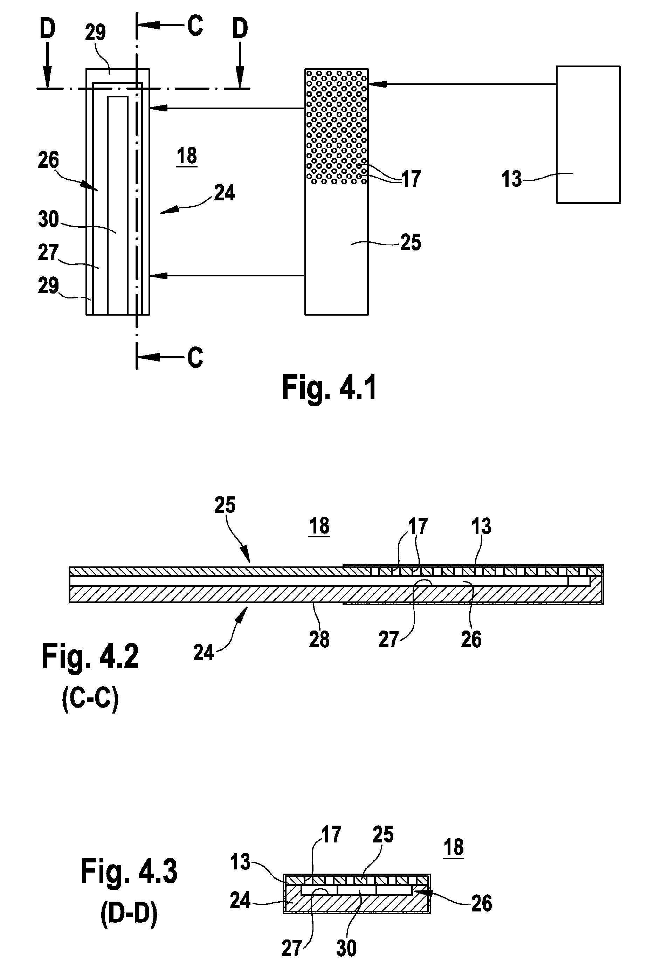

Figuren 4.1 bis 4.3 zeigen schematisch eine vierte Ausführungsform eines erfindungsgemäßen Mikrodialysekatheters mit einem Träger in Form eines ebenen Substrats mit Abdeckung.

Gemäß einer weiteren Ausführungsform der vorliegenden Erfindung ist der Träger des Mikrodialysekatheters ein ebenes Substrat mit einer Abdeckung. Figur 4.1 zeigt eine Draufsicht auf die einzelnen Lagen eines solchen Mikrodialysekatheters, Figur 4.2 einen Längsschnitt (entlang C - C in Figur 4.1) und Figur 4.3 einen Querschnitt (entlang D - D in Figur 4.1) durch einen solchen Mikrodialysekatheter auf der Basis eines ebenen Substrats 24 mit einer Abdeckung 25. Die Schnitte sind dabei in den Figuren 4.2 und 4.3 jeweils in vergrößerter Darstellung gezeigt.

Das ebene Substrat 24 enthält als Leiter eine Kanalstruktur 26 zum Zuführen einer Perfusionsflüssigkeit und zum Abführen eines Dialysats. Die Kanalstruktur 26 wird durch eine äußere Wand 29 und einen inneren Steg 30 begrenzt und durch die Abdeckung 25 des ebenen Substrats 24 geschlossen, wobei die Abdeckung 25 eine Vielzahl von Öffnungen 17 aufweist, die durch einen für einen Analyten per Diffusion durchlässigen, porenfreien Überzug 13 überspannt sind und die mit der Kanalstruktur 26 fluidisch verbunden sind.

Zur Herstellung eines solchen Mikrodialysekatheters wird in ein ebenes Substrat 24 eine Kanalstruktur 26 eingebracht, welche die Zuleitung einer Perfusionsflüssigkeit und die Ableitung des Dialysats ermöglicht. Die Kanalstruktur 26 wird durch Abdeckung 25 einer an der Oberfläche des Substrats 24 liegenden Rinne 27 mit einer Abdeckplatte oder Abdeckfolie allseitig abgeschlossen. In die abdeckende Platte/Folie 25 werden Öffnungen 17 eingebracht, welche eine fluidische Verbindung zwischen dem den Mikrodialysekatheter umgebenden Außenraum 18 und der Kanalstruktur 26 herstellen.

Die mit den Öffnungen 17 versehene Abdeckung 25 wird außen mit einem für den Analyten per Diffusion durchlässigen, porenfreien Überzug 13 versehen. Der Überzug 13 bedeckt die Außenwand 28 des Substrates 24 und der Abdeckung 25. Die eingebrachten Öffnungen 17 werden von dem Überzug 13 überspannt. Das mit dem genannten Überzug 13 versehene Substrat 24 wird gegebenenfalls mit einem weiteren (nicht dargestellten) biokompatiblen Überzug versehen, welcher von dem Analyten durchströmt werden kann.

### Bezugszeichenliste

- 1: Träger
- 2: Innenraum
- 3: Öffnung
- 4: Abdichtung
- 5: erster fluidischer Leiter/erste innere Röhre
- 6a: offenes Ende des ersten fluidischen Leiters
- 6b: seitliche Öffnung in dem ersten fluidischen Leiter
- 7: Außenraum
- 8: zweiter fluidischer Leiter/zweite innere Röhre
- 9: offenes Ende des zweiten fluidischen Leiters
- 10: distales Ende
- 11: zum Implantieren vorgesehener Bereich
- 12: Röhre
- 13: Überzug
- 14: Außenwand der Mehrlumenröhre
- 15: Trennwand
- 16: Innenlumen
- 17: Öffnungen
- 18: Außenraum
- 19: Innenraum
- 20: erstes Innenlumen
- 21: zweites Innenlumen
- 22: Dichtung
- 23: fluidische Verbindung
- 24: ebenes Substrat
- 25: Abdeckung
- 26: Kanalstruktur
- 27: Rinne
- 28: Außenwand des Substrats
- 29: äußere Wand
- 30: innerer Steg
- 31: Mehrlumenröhre

## Patentansprüche

1. Verfahren zur Herstellung eines Mikrodialysekatheters, **gekennzeichnet durch** die Schritte
a) Bereitstellen eines Trägers (1, 24, 31), der mindestens einen Leiter (5, 8, 16, 26) zum Zuführen einer Perfusionsflüssigkeit und zum Abführen eines Dialysats enthält und der einen zum Implantieren vorgesehenen Bereich (11) aufweist, in dem mindestens eine fluidisch mit dem Leiter (5, 8, 16, 26) verbundene Öffnung (3, 17) enthalten ist, und
b) Überziehen des Trägers (1, 24, 31) mit einem für einen Analyten per Diffusion durchlässigen, porenfreien Überzug (13), der den zum Implantieren vorgesehenen Bereich (11) von außen zumindest teilweise bedeckt, mit dem Träger (1, 24, 31) stoffschlüssig verbunden ist und die mindestens eine Öffnung (3, 17) überspannt.

2. Verfahren gemäß Anspruch 1, **dadurch gekennzeichnet, dass** der Träger (1, 24, 31) in dem zum Implantieren vorgesehenen Bereich (11) mit einer Polymerlösung überzogen wird, um den Überzug (13) herzustellen.

3. Verfahren gemäß Anspruch 1 oder 2, **dadurch gekennzeichnet, dass** der Träger (1, 24, 31) zum Herstellen des Überzugs (13) in eine Polymerlösung getaucht oder mit einer Polymerlösung besprüht wird, oder dass der Überzug (13) durch Rakeln oder Rotationsbeschichtung mit einer Polymerlösung erzeugt wird.

4. Verfahren gemäß einem der Ansprüche 1 bis 3, **gekennzeichnet durch** Überziehen des Trägers (1, 24, 31) mit einer Polymerlösung, die **durch** Erwärmen, **durch** Bestrahlen mit elektromagnetischer Strahlung oder **durch** Kontaktieren mit Wasser ausgehärtet wird.

5. Verfahren gemäß einem der Ansprüche 1 bis 4, **gekennzeichnet durch** Bedecken des Überzugs (13) mit einer Schicht aus einem biokompatiblen, porösen Material.

6. Mikrodialysekatheter, hergestellt durch ein Verfahren gemäß einem der Ansprüche 1 bis 5, umfassend einen Träger (1, 24, 31), der mindestens einen Leiter (5, 8, 16, 26) zum Zuführen einer Perfusionsflüssigkeit und zum Abführen eines Dialysats enthält, und der einen zum Implantieren vorgesehenen Bereich (11) aufweist, in dem mindestens eine fluidisch mit dem Leiter (5, 8, 16, 26) verbundene Öffnung (3, 17) enthalten ist, wobei der Träger (1, 24, 31) einen für einen Analyten per Diffusion durchlässigen, porenfreien Überzug (13) aufweist, der den zum Implantieren vorgesehenen Bereich (11) zumindest teilweise von außen bedeckt, mit dem Träger (1, 24, 31) stoffschlüssig verbunden ist und die mindestens eine Öffnung (3, 17) überspannt.

7. Mikrodialysekatheter gemäß Anspruch 6, **dadurch gekennzeichnet, dass** der Überzug (13) eine Beschichtung aus einem biokompatiblen Material aufweist.

8. Mikrodialysekatheter gemäß einem der Ansprüche 6 oder 7, **dadurch gekennzeichnet, dass** der Träger (1) eine Röhre (12) ist, deren distales Ende (10) verschlossen ist und die mindestens eine seitliche Öffnung (3) aufweist, die durch einen für einen Analyten per Diffusion durchlässigen Überzug (13) überspannt ist, wobei die Röhre (12) einen longitudinal angeordneten Innenraum (2) enthält, in dem mindestens ein fluidischer Leiter (5, 8) zum Zuführen einer Perfusionsflüssigkeit und zum Abführen eines Dialysats vorgesehen ist, wobei der fluidische Leiter (5, 8) mit der mindestens einen seitlichen Öffnung (3) fluidisch verbunden ist.

9. Mikrodialysekatheter gemäß Anspruch 8, **dadurch gekennzeichnet, dass** als fluidische Leiter (5, 8) im Innenraum (2, 19) der Röhre durch eine Trennwand (15) voneinander getrennte Innenlumen (16) oder zwei innere Röhren (5, 8) vorgesehen sind, die fluidisch miteinander verbunden sind.

10. Mikrodialysekatheter gemäß einem der Ansprüche 6 oder 7, **dadurch gekennzeichnet, dass** der Träger (1) ein ebenes Substrat (24) und eine Abdeckung (25) umfasst, wobei das Substrat (24) als Leiter eine Kanalstruktur (26) zum Zuführen einer Perfusionsflüssigkeit und zum Abführen eines Dialysats enthält, wobei die Kanalstruktur (26) durch die Abdeckung (25) des ebenen Substrats (24) geschlossen ist, und wobei der Träger (1) mindestens eine Öffnung (17) aufweist, die durch einen für einen Analyten per Diffusion durchlässigen, porenfeien Überzug (13) überspannt ist und die mit der Kanalstruktur (26) fluidisch verbunden ist.

## Geänderte Patentansprüche

### Geänderte Patentansprüche gemäss Regel 137(2) EPÜ.

**1.** Verfahren zur Herstellung eines Mikrodialysekatheters, **gekennzeichnet durch** die Schritte
a) Bereitstellen eines Trägers (1, 24, 31), der mindestens einen Leiter (5, 8, 16, 26) zum Zuführen einer Perfusionsflüssigkeit und zum Abführen eines Dialysats enthält und der einen zum Implantieren vorgesehenen Bereich (11) aufweist, in dem mindestens eine fluidisch mit dem Leiter (5, 8, 16, 26) verbundene Öffnung (3, 17) enthalten ist, und
b) Überziehen des Trägers (1, 24, 31) mit einem für einen Analyten per Diffusion durchlässigen, porenfreien Überzug (13), der den zum Implantieren vorgesehenen Bereich (11) von außen zumindest teilweise bedeckt, mit dem Träger (1, 24, 31) stoffschlüssig verbunden ist und die mindestens eine Öffnung (3, 17) überspannt, wobei der wobei der Überzug (13) zum Überziehen des Trägers (1, 24, 31) direkt auf den Träger (1, 24, 31) abgeschieden wird.

**2.** Verfahren gemäß Anspruch 1, **dadurch gekennzeichnet, dass** der Träger (1, 24, 31) in dem zum Implantieren vorgesehenen Bereich (11) mit einer Polymerlösung überzogen wird, um den Überzug (13) herzustellen.

**3.** Verfahren gemäß Anspruch 1 oder 2, **dadurch gekennzeichnet, dass** der Träger (1, 24, 31) zum Herstellen des Überzugs (13) in eine Polymerlösung getaucht oder mit einer Polymerlösung besprüht wird, oder dass der Überzug (13) durch Rakeln oder Rotationsbeschichtung mit einer Polymerlösung erzeugt wird.

**4.** Verfahren gemäß einem der Ansprüche 1 bis 3, **gekennzeichnet durch** Überziehen des Trägers (1, 24, 31) mit einer Polymerlösung, die **durch** Erwärmen, **durch** Bestrahlen mit elektromagnetischer Strahlung oder **durch** Kontaktieren mit Wasser ausgehärtet wird.

**5.** Verfahren gemäß einem der Ansprüche 1 bis 4, **gekennzeichnet durch** Bedecken des Überzugs (13) mit einer Schicht aus einem biokompatiblen, porösen Material.

**6.** Mikrodialysekatheter, hergestellt durch ein Verfahren gemäß einem der Ansprüche 1 bis 5, umfassend einen Träger (1, 24, 31), der mindestens einen Leiter (5, 8, 16, 26) zum Zuführen einer Perfusionsflüssigkeit und zum Abführen eines Dialysats enthält, und der einen zum Implantieren vorgesehenen Bereich (11) aufweist, in dem mindestens eine fluidisch mit dem Leiter (5, 8, 16, 26) verbundene Öffnung (3, 17) enthalten ist, wobei der Träger (1, 24, 31) einen für einen Analyten per Diffusion durchlässigen, porenfreien Überzug (13) aufweist, der den zum Implantieren vorgesehenen Bereich (11) zumindest teilweise von außen bedeckt, mit dem Träger (1, 24, 31) stoffschlüssig verbunden ist und die mindestens eine Öffnung (3, 17) überspannt, wobei der Überzug (13) zum Überziehen des Trägers (1, 24, 31) direkt auf den Träger (1, 24, 31) abgeschieden ist.

**7.** Mikrodialysekatheter gemäß Anspruch 6, **dadurch gekennzeichnet, dass** der Überzug (13) eine Beschichtung aus einem biokompatiblen Material aufweist.

**8.** Mikrodialysekatheter gemäß einem der Ansprüche 6 oder 7, **dadurch gekennzeichnet, dass** der Träger (1) eine Röhre (12) ist, deren distales Ende (10) verschlossen ist und die mindestens eine seitliche Öffnung (3) aufweist, die durch einen für einen Analyten per Diffusion durchlässigen Überzug (13) überspannt ist, wobei die Röhre (12) einen longitudinal angeordneten Innenraum (2) enthält, in dem mindestens ein fluidischer Leiter (5, 8) zum Zuführen einer Perfusionsflüssigkeit und zum Abführen eines Dialysats vorgesehen ist, wobei der fluidische Leiter (5, 8) mit der mindestens einen seitlichen Öffnung (3) fluidisch verbunden ist.

**9.** Mikrodialysekatheter gemäß Anspruch 8, **dadurch gekennzeichnet, dass** als fluidische Leiter (5, 8) im Innenraum (2, 19) der Röhre durch eine Trennwand (15) voneinander getrennte Innenlumen (16) oder zwei innere Röhren (5, 8) vorgesehen sind, die fluidisch miteinander verbunden sind.

**10.** Mikrodialysekatheter gemäß einem der Ansprüche 6 oder 7, **dadurch gekennzeichnet, dass** der Träger (1) ein ebenes Substrat (24) und eine Abdeckung (25) umfasst, wobei das Substrat (24) als Leiter eine Kanalstruktur (26) zum Zuführen einer Perfusionsflüssigkeit und zum Abführen eines Dialysats enthält, wobei die Kanalstruktur (26) durch die Abdeckung (25) des ebenen Substrats (24) geschlossen ist, und wobei der Träger (1) mindestens eine Öffnung (17) aufweist, die durch einen für einen Analyten per Diffusion durchlässigen, porenfeien Überzug (13) überspannt ist und die mit der Kanalstruktur (26) fluidisch verbunden ist.
